Europäisches Patentamt

European Patent Office

Office européen des brevets

(11) Veröffentlichungsnummer: **0 512 238 A1**

# EUROPÄISCHE PATENTANMELDUNG

(21) Anmeldenummer: **92105241.1**

(22) Anmeldetag: **27.03.92**

(51) Int. Cl.5: **G01N 33/18**, G01N 21/37

(30) Priorität: **10.05.91 DE 4115425**

(43) Veröffentlichungstag der Anmeldung:
**11.11.92 Patentblatt 92/46**

(84) Benannte Vertragsstaaten:
**DE FR GB IT NL**

(71) Anmelder: **Hartmann & Braun**
**Akfiengesellschaft**
**Gräfstrasse 97**
**W-6000 Frankfurt am Main 90(DE)**

(72) Erfinder: **Fabinski, Walter**
**An der Landwehr 70**
**W-6239 Kriftel(DE)**
Erfinder: **Hübschmann, Peter**
**Fritz-von-Leonhardi-Strasse 38**
**W-6369 Nidderau 1(DE)**
Erfinder: **Schlau, Peter**
**Zum Jungenstrasse 16**
**W-6000 Frankfurt 1(DE)**
Erfinder: **Wolff, Christian**
**Frankfurter Strasse 2a**
**W-6367 Karben 2(DE)**

(54) **Verfahren zur Messung des Gesamtgehaltes an organischem Kohlenstoff und an Stickstoff in Wasser.**

(57) Die Anmeldung betrifft ein Verfahren zur Messung des Gesamtgehaltes an organischem Kohlenstoff (TOC) und an Stickstoff (TN) in Wasser. Der TOC-Wert wird korrekt als Summe der flüssigen, gelösten und festen Stoffe einer Probe des Wassers ermittelt. Die Vorrichtung besteht aus einem NDIR-Gasanalysator zur gleichzeitigen Messung der Konzentration der Gaskomponenten $CO_2$ und NO mit einem Phasentrenner T, einem thermischen Reaktor OX, einem Kühler K, zwei Ventilen MV1 und MV2 sowie zwei Verstärkern A1 und A2 für die pneumatischen Signale der Empfänger E1 und E2 mit einer Anzeige für die gemessenen Konzentrationen TOC und TN. Die Probe wird im Phasentrenner aufgespalten in einen gasförmigen und in einen flüssigen Teil. Der gasförmige Teil, der im wesentlichen den anorganischen Anteil an Kohlenstoff, den TIC-Anteil, enthält in der Form von $CO_2$-Gas, wird in einem Kühler so weit abgekühlt, daß sich ein wesentlicher Anteil seines Wasserdampfgehaltes im Kühler durch Kondensation abscheidet. Der getrocknete gasförmige Teil wird als Vergleichsgas über die Vergleichsküvette geleitet. Diese Maßnahme dient dazu, den TIC-Anteil der Probe zu kompensieren und die nicht zu vermeidende Wasserdampf-Querempfindlichkeit bei der Messung der $CO_2$- und der NO-Konzentration zu kompensieren. Der aus der Vergleichsküvette austretende gasförmige Teil wird dem flüssigen Teil der Probe wieder vollständig zugeführt und mit diesem in einem thermischen Reaktor oxidiert. Die dabei entstehende Gasprobe wird über den Kühler K mit Wasserdampf der gleichen Temperatur wie die des Vergleichsgases V beladen und als Meßgas M der Meßküvette MK zugeführt wird, wobei das Meßgas M den gesamten Kohlendioxidanteil und den gesamten Stickoxidanteil der Gasprobe beinhaltet.

Die Erfindung betrifft ein Verfahren zur Messung des Gesamtgehaltes an organischem Kohlenstoff und an Stickstoff in Wasser nach dem Oberbegriff des Anspruches 1.

Im folgenden bedeutet

TOC (Total Organic Carbon) der gesamte im Meßwasser vorhandene Kohlenstoff in Form von organischen Verbindungen;

TN (Total Nitrogin) der gesamte im Meßwasser vorhandene Stickstoff;

TIC (Total Inorganic Carbon) der gesamte im Meßwasser vorhandene Kohlenstoff in Form von anorganischen Verbindungen;

TC (Total Carbon) der gesamte im Wasser vorhandene Kohlenstoff.

Der organische Anteil von Kohlenstoff TOC ist ein Sauerstoffzehrer und damit interessant für die Beurteilung von Wässern. Die Grundlage für die Bestimmung des TOC-Wertes ist in der DIN 38409 (H) Teil 3 festgelegt. Häufig wird in der Praxis an Stelle des korrekten Wertes nur ein Teil, nämlich der des gelösten TOC-Anteils erfaßt. Im Wasser können häufig auch flüchtige organische Bestandteile vorhanden sein, die bei der Trennung der Probe von anorganischen Bestandteilen verlorengehen. Damit wird der TOC-Wert nicht vollständig erfaßt.

Neben dem TOC-Wert ist der Anteil von gebundenem Stickstoff TN interessant; er erlaubt eine Aussage zur Belastung der Wässer mit Stickstoffverbindungen aus natürlichen und industriellen Emittenten. Ein Vorschlag zur Messung ist in dem DIN-Entwurf 38 409 Teil 27 niedergelegt.

TOC-Bestimmungen sind bekannt. Man mißt TC und TIC und berechnet durch Differenzbildung TOC; (DE-OS 28 11 135, DE-OS 24 58 143, DE-AS 22 60 295, DE-OS 23 22 293, EP-PS 01 50 923).

In der DE-OS 39 09 240 ist ein Verfahren zum kontinuierlichen und quantitativen Bestimmen von organischen und anorganischen Kohlenstoffverbindungen in Wasser beschrieben, bei dem das zu untersuchende Wasser in einem Ausgasegefäß angesäuert wird. Durch das Wasser im Ausgasegefäß wird ein Transport-Gasstrom geleitet. Wasser und Transportgas gelangen aus dem Ausgasegefäß in einen Zersetzungsreaktor. Der $CO_2$-Gehalt des Transportgases wird hinter dem Ausgasungsgefäß oder hinter dem Zersetzungsreaktor bestimmt. Die Anteile an organischen und organischen C-Verbindungen werden getrennt voneinander oder als Summe bestimmt.

In dem Aufsatz von F. Ehrenberger, "Zur Bestimmung von Sauerstoffbedarfs- und Kohlenstoff-Kennzahlen in der Wasserqualitätsbestimmung", GIT Fachz. Lab. 23.Jg. 8/79, S. 738-747, werden verschiedene Methoden zur TOC-Bestimmung beschrieben, die auf der naßchemischen oder thermischen Umsetzung der organischen Inhaltsstoffe und der quantitativen Oxidation des organisch gebundenen Kohlenstoffes zu Kohlendioxid beruhen. Der $CO_2$-Gehalt wird auf chemischem oder physikalischem Wege bestimmt. Die gleichzeitige Bestimmung von Stickstoff sehen die beschriebenen Methoden nicht vor.

Im Einzelfall wird einer TOC-Messung eine TN-Zusatzmessung durch Beistellung eines entsprechenden Analysators hinzugefügt, was aufwendig ist und unerwünschte Verzugszeiten der Anzeige schafft (DE-OS 26 21 616). Die Kalibrierung erfolgt häufig von Hand mit einem hochreinem Nullwasser für die Nullpunkt-Einstellung und mit einer Prüflösung für die Empfindlichkeit. Es werden auch prinzipbedingte Meßfehler in Kauf genommen: so treten Abweichungen von der vorgegebenen Empfindlichkeit auf, wenn sich eine $CO_2$-Grundlast verändert.

In der DE-OS 39 37 141 ist ein NDIR-Gasanalysator zur gleichzeitigen Messung der Konzentration mehrerer Komponenten einer Gasprobe beschrieben, der zur Bestimmung der beiden Gaskomponenten Kohlendioxid und Stickoxid geeignet ist.

Der Erfindung liegt die Aufgabe zugrunde, ein Verfahren zur gleichzeitigen Messung des TOC- und des TN-Gehaltes im Wasser anzugeben, die die gelösten wie auch die flüchtigen Bestandteile erfaßt, die Nullpunktverschiebungen und Empfindlichkeitsänderungen auf Grund von $CO_2$-Änderungen bei der TOC-Messung sowie Querempfindlichkeiten gegenüber Wasserdampfanteilen bei der TN-Messung berücksichtigt und eine Nullpunktkalibrierung ohne Nullwasser ermöglicht.

Die gestellte Aufgabe wird durch die im Anspruch 1 angegebenen Maßnahmen gelöst. Eine Probe des zu untersuchenden Wassers wird in einem Phasentrenner aufgespalten in einen gasförmigen und in einen flüssigen Teil. Der gasförmige Teil, der im wesentlichen den anorganischen Anteil an Kohlenstoff enthält, dem TIC-Anteil, in der Form von $CO_2$-Gas und teilweise auch Kohlenstoffanteile in der Form von leicht flüchtigen Kohlenwasserstoff-Verbindungen, wird in einem Kühler auf etwa +4°C so weit abgekühlt, daß ein überwiegender Anteil seines Wasserdampfgehaltes im Kühler durch Kondensation abgeschieden wird. Der so getrocknete gasförmige Teil gelangt als Vergleichsgas in die Vergleichsküvette eines nach dem Stoffvergleich arbeitenden NDIR-Gasanalysators. Diese Maßnahme dient dazu, den TIC-Anteil der Probe zu kompensieren und die nicht zu vermeidende Wasserdampf-Querempfindlichkeit bei der Messung der $CO_2$- und der NO-Konzentration zu kompensieren. Der aus der Vergleichsküvette austretende gasförmige Teil wird dem flüssigen Teil der Probe wieder vollständig zugeführt und mit diesem zusammen in einem thermischen Reaktor oxidiert. Die Oxidation der Probe erfolgt sowohl für den flüchtigen als auch für den im Wasser gelösten Kohlenstoffanteil zu $CO_2$ und für den Stickstoffanteil zu NO.

Der so gewonnenen Gasprobe, die den gesamten Kohlenstoffanteil und den gesamten Stickstoffanteil der Probe enthält, wird in dem Kühler durch Kondensation bei der gleichen Temperatur wie die des Vergleichsgases der Wasserdampf entzogen und als Meßgas der Meßküvette des NDIR-Gasanalysators zugeführt. Im ersten auf $CO_2$ sensibilisierten Empfänger entsteht ein pneumatisches Signal, welches dem Konzentrationswert TC minus TIC und damit dem gesuchten TOC-Wert entspricht. Im nachfolgenden auf Stickstoff sensibilisierten Empfänger entsteht ein pneumatisches Signal, welches der Differenz aus dem wasserdampfbeladenen Stickoxid-Anteil des Meßgases und dem Wasserdampfanteil des Vergleichsgases, also dem gesuchten Stickstoffanteil TN in der Probe entspricht.

Zum Abgleich des Nullpunktes und der Empfindlichkeit des Analysators wird die dem Phasentrenner entnommene Gasprobe sowohl durch den Meß- als auch durch den Vergleichskanal geleitet. Mit dieser Einstellung werden Nullpunktverschiebungen der den Empfängern nachgeschalteten Verstärkern auf Grund von $CO_2$-Offsetänderungen oder die Querempfindlichkeit auslösende $H_2O$-Anteile wie auch Alterungen und Verschmutzungen des Gasanalysators abgeglichen. Durch Einschieben einer Kalibrierküvette in den Strahlengang zwischen den Küvetten und den Empfängern wird schließlich der Endpunkt der beiden Verstärker auf die Konzentrationswerte eingestellt, die durch die in der Kalibrierküvette eingeschlossenen Prüfgas vorgegeben sind. Diese Abgleichmethode gewährleistet, daß auch kleine Meßbereiche mit ausreichender Stabilität realisierbar sind; sie kann zudem unter Verzicht auf externe Prüfgas aus Vorratsflaschen durchgeführt werden.

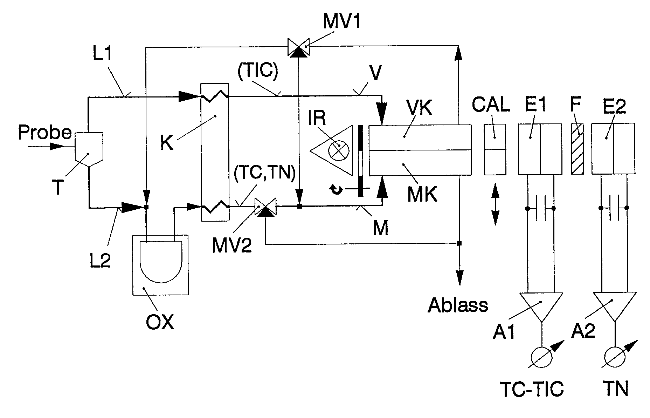

Ein Ausführungsbeispiel der Erfindung wird anhand eines in der Zeichnung dargestellten Blockschaltbildes näher erläutert.

Die Vorrichtung besteht aus einem bekannten NDIR-Gasanalysator zur gleichzeitigen Messung der Konzentration der Gaskomponenten $CO_2$ und NO mit zwei nebeneinanderliegenden Küvetten, der Meßküvette MK für das Meßgas M und der Vergleichsküvette VK für das Vergleichsgas V. Die Küvetten MK und VK werden von modulierten Lichtstrahlen eines Infrarotstrahlers IR durchstrahlt, die nach teilweiser Absorption in den Küvetten auf pneumatische Empfänger E1 und E2 fallen. Die Empfänger weisen zwei in Strahlungsrichtung hintereinander angeordnete Kammern auf, die jeweils mit einer der zu bestimmenden Komponente der Gasprobe gefüllt sind und zwar Empfänger E1 mit der Komponente $CO_2$ und Empfänger E2 mit der Komponente NO.

Zwischen den Empfängern E1 und E2 ist ein Filter F angeordnet, welches für die Lichtstrahlen in Bereich der Strahlungsabsorption der Komponente NO der Gasprobe transparent ist. Außerdem ist in den Strahlengang zwischen die Küvetten VK,MK und dem ersten Empfänger E1 eine Kalibrierküvette CAL mit zwei nebeneinanderliegenden Kammern einschiebbar. Ihre Kammern sind gefüllt mit einem Kalibriergas, beispielsweise mit $CO_2$ und NO im Meßstrahlengang und mit im Vergleichsstrahlengang absorptionsneutralen Stickstoff $N_2$ zur Einstellung der Empfindlichkeit des Gasanalysators für die zu messenden Gaskomponenten $CO_2$ und NO.

Die Vorrichtung besteht ferner aus einem Phasentrenner T, einem thermischen Reaktor OX, einem Kühler K, zwei Ventilen MV1 und MV2 sowie zwei Verstärkern A1 und A2 für die pneumatischen Signale der Empfänger E1 und E2 mit einer Anzeige für die gemessenen Konzentrationen TC-TIC und TN.

Eine Probe des zu untersuchenden Wassers gelangt in den Phasentrenner T, welcher den anorganischen Kohlenstoffanteil TIC von der Probe trennt, in $CO_2$-Gas umsetzt und über die Leitung L1 dem Kühler K zuführt. Hier wird die Hauptmenge des Wasserdampfes aus dem $CO_2$-Gas durch Kondensation entfernt. Das derart bei etwa 4°C getrocknete $CO_2$-Gas gelangt als Vergleichsgas V in die Vergleichsküvette VK, durchströmt diese und gelangt von dort über das nicht aktivierte Ventil MV1 in den thermischen Reaktor OX, wo es zusammen mit dem aus dem Phasentrenner T über die Leitung L2 eingespeisten flüssigen Teil der Probe oxidiert wird zu $CO_2$ und NO. Das gasförmige Oxidationsprodukt, die Gasprobe, enthält den gesamten Kohlenstoff- und Stickstoffanteil der wässrigen Probe. Im Kühler K wird der Gasprobe durch Kondensation bei der gleichen Temperatur von etwa 4°C der wesentliche Teil des Wasserdampfes entzogen. Die getrocknete Gasprobe gelangt über das nicht aktivierte Ventil MV2 als Meßgas M in die Meßkammer M des Gasanalysators.

Da das Vergleichsgas $CO_2$ enthält, dessen Konzentration dem aktuellen anorganischen Kohlenstoffanteil TIC der Probe entspricht, entsteht im ersten für $CO_2$ empfindlichen Empfänger E1 ein pneumatisches Signal, welches der Differenz aus dem Konzentrationswert für TC minus dem Konzentrationswert für TIC entspricht. Dieser Differenzwert TC-TIC wird über den Verstärker A1 zur Anzeige gebracht. Gleichzeitig entsteht im Empfänger E2 ein pneumatisches Signal, welches dem Konzentrationswert des gesuchten Stickstoffanteiles TN entspricht.

Die bei der Messung von NO auftretenden Querempfindlichkeiten gegenüber Wasserdampf lassen sich nur teilweise durch Kondensation der Gase im vorgeschalteten Kühler K beseitigen. Mit der Maßnahme, das Meßgas M und das Vergleichsgas V gleichzeitig über den selben Kühler zu leiten und bei der gleichen Temperatur abzukühlen, gelingt es, Meß- und Vergleichsgas auf den gleich niedrigen Wasserdampfgehalt einzustellen, so daß sich die in beiden Küvetten auftretenden Querempfindlichkeiten kompensieren und das pneumatische Signal im Empfänger E2 dem unverfälschten NO-Konzentrationswert der Gasprobe entspricht.

Der Nullpunkt und die Empfindlichkeiten der Meßvorrichtung sind verschiedenen Einflüssen unterworfen. Dazu gehören Alterungen und Wasserdampf-Querempfindlichkeiten des Gasanalysators sowie einem durch das Meßprinzip mit strömendem Vergleichsgas bedingten Empfindlichkeitseinfluß durch den anorganischen $CO_2$-Anteil. Letzterer kann die Empfindlichkeit je nach Konzentration bis zu einigen 100% beeinflussen. Um diese Einflußeffekte zu eliminieren, wird die Meßvorrichtung in vorgegebenen Zeitintervallen wie folgt abgeglichen:

Die dem Phasentrenner T entnommene Gasprobe wird über die Leitung L1 und einen Kühlweg des Kühlers K der Vergleichsküvette VK und durch Aktivierung der Ventile MV1 und MV2 der Meßküvette MK und dem Ablass zugeführt. Das Ventil MV2 kann auch vor dem Kühler K geschaltet werden so, daß die Gasprobe noch über den Kühlweg des Kühlers fließt. Mit dieser Einstellung werden die den Nullpunkt verändernden Einflüsse mit Hilfe elektronischer Mittel in den Verstärkern A1 und A2 abgeglichen. Danach wird die Kalibrierküvette CAL in den Strahlengang zwischen den Küvetten VK und MK und den Empfängern E1 und E2 geschoben und die Empfindlich-keit für den $CO_2$- und den NO-Kanal entsprechend der in der Kalibrierküvette CAL befindlichen Kalibriergase eingestellt. Diese Einstellung berücksichtigt neben Gerätealterungen und Verschmutzungen auch den Einfluß der aktuellen anorganischen $CO_2$-Konzentration und zeigt den TOC-Wert der Probe als Summe aus den flüchtigen, gelösten und festen Stoffen korrekt an. Vorteilhaft ist, daß keine Nullflüssigkeit oder Prüfgas für diese Abgleichmaßnahme benötigt wird.

Außer dem oben beschriebenen Vorgang zur Justierung der Meßvorrichtung ist noch eine Kalibrierung der Gesamtmeßeinrichtung durchzuführen. Hierbei werden auch Module wie Pumpen berücksichtigt. Diese Kalibrierung wird ohne Nullflüssigkeit durchgeführt. Die Kalibrierung des Nullpunktes und der Empfindlichkeit erfolgt mit einer Kalibrierflüssigkeit, die eine bekannte Konzentration an organischem Kohlenstoff und an Stickstoffverbindungen enthält. Sie wird als Probe dem Phasentrenner T zugeführt. In einem ersten Schritt wird der Nullpunkt und die Empfindlichkeit der Vorrichtung nach der oben beschriebenen Justierung durch Aktivierung der Ventile MV1 und MV2 eingestellt. In einem zweiten Schritt werden diese Ventile deaktiviert. Die Empfänger E1 und E2 liefern jeweils ein pneumatisches Signal, welches der bekannten Konzentration an organischem Kohlenstoff und an Stickstoffverbindungen entspricht. Mit den so erzeugten Signalen wird die Empfindlichkeit der nachgeschalteten Verstärker A1 und A2 mit elektronischen Mitteln eingestellt. Nach Abschluß des Kalibriervorganges wird die Vorrichtung wieder auf den Meßbetrieb zur Messung der dem zu untersuchenden Wasser entnommenen Proben zurückgestellt. Dieser Vorgang kann mit nicht dargestellten Ventilen per Hand oder automatisch ausgeführt werden.

Unmittelbar an die Kalibrierung der Meßvorrichtung kann sich ein Abgleichvorgang mit der Probe des Wassers, wie oben beschrieben, anschließen, um den Bezug zum organischen $CO_2$-Gehalt der Probe herzustellen.

Es ist zweckmäßig, den Abgleichvorgang häufiger, beispielsweise alle zwei bis zwölf Stunden durchzuführen, während der Kalibriervorgang mit der Kalibrierflüssigkeit je nach Meßbedingungen nur alle 2 bis 24 Tage oder in noch längeren Zeitintervallen durchgeführt werden sollte.

**Patentansprüche**

1. Verfahren zur Messung des Gesamtgehaltes an organischem Kohlenstoff (TOC) und an Stickstoff (TN) in Wasser
   - durch Verdampfen einer dem Wasser entnommenen Probe und Oxydieren des organischen Kohlenstoffes zu Kohlendioxid und des Stickstoffes zu Stickoxid in einem thermischen Reaktor, wobei die Messung auf der Grundlage der aus der Probe gebildeten Gasprobe aus Kohlendioxid ($CO_2$) und Stickoxid (NO) vorgenommen wird,
   - unter Verwendung eines NDIR-Gasanalysators zur gleichzeitigen Messung der Gaskomponenten Kohlendioxid und Stickoxid
     - mit zwei nebeneinanderliegenden Küvetten, denen die Gasprobe bzw. ein Vergleichsgas zugeführt werden und die von modulierten Lichtstrahlen eines Infrarotstrahlers durchdrungen werden,
     - die Lichtstrahlen nach teilweiser Absorption in den Küvetten auf hinter den Küvetten angeordnete pneumatische Empfänger fallen, jeder der Empfänger zwei in Strahlungsrichtung hintereinander angeordnete Kammern aufweisen, welche beide jeweils mit derselben zu bestimmenden Komponente der Gasprobe gefüllt sind,
- zwischen den Empfängern ein Strahlungsfilter eingesetzt ist, welches für die Lichtstrahlen im Bereich der Strahlungsabsorption der Komponente Stickoxid (NO) der Gasprobe transparent ist und
- mit einer Kalibrierküvette mit zwei Kammern, die in den Strahlengang zwischen die Küvetten und die Empfänger geschoben werden und die gefüllt sind mit einem Kalibriergas im Meßstrahlengang, vorzugsweise $CO_2$ und NO, sowie mit einem neutralen Gas im Vergleichsstrahlengang, vorzugsweise $N_2$, zur Einstellung der Empfindlichkeit des Gasanalysators für die zu messenden Gaskomponenten ($CO_2$, NO)

dadurch gekennzeichnet, daß
- als Vergleichsgas (V) der mit Hilfe eines Phasentrenners (T) aus der Probe abgeschiedene und über einen Kühler (K) mit Wasserdampf beladene anorganische Kohlendioxidanteil der Gasprobe dient,
- das Vergleichsgas (V) nach Durchlaufen der Vergleichsküvette (VK) der zu verdampfenden und zu oxidierenden Probe zugeführt wird,
- die in der Probe vorhandenen Kohlenstoff- und Stickstoffanteile im thermischen Reaktor (OX) oxidiert erden, die dabei entstehende Gasprobe über den Kühler (K) mit Wasserdampf der gleichen Temperatur wie die des Vergleichsgases (V) beladen und als Meßgas (M) der Meßküvette (MK) zugeführt wird, wobei das Meßgas (M) den gesamten Kohlendioxidanteil und den gesamten Stickoxidanteil der Gasprobe beinhaltet,
- das im ersten Empfänger (E1) entstehende pneumatische Signal, welches der Differenz aus dem gesamten Kohlenstoffanteil (TC) und dem anorganischen Kohlenstoffanteil (TIC) und somit dem organischen Kohlenstoffanteil (TOC) der Probe entspricht, mit einem ersten elektrischen Verstärker (A1) gemessen wird,
- das im zweiten auf Stickoxid sensibilisierten Empfänger (E2) entstehende pneumatische Signal, welches der Differenz aus dem wasserdampfbeladenen Stickoxidanteil des Meßgases (M) und dem Wasserdampfanteil des Vergleichsgases (V) und somit dem Stickstoffanteil (TN) der Probe entspricht, mit einem zweiten elektrischen Verstärker (A2) gemessen wird,
- zur Berücksichtigung des anorganischen Kohlendioxidanteils, der als störender Untergrund in der Probe vorhanden ist, das Vergleichsgas (V) mit dem aktuellen anorganischen Kohlendioxidanteil gleichzeitig durch die Meß- (MK) und die Vergleichsküvette (VK) geleitet wird, wobei in einem ersten Schritt der Nullpunkt des NDIR-Gasanalysators für den TOC-Wert und den TN-Wert eingestellt wird und in einem zweiten Schritt die Kalibrierküvette (CAL) in den Strahlengang geschoben wird und die Empfindlichkeit des ersten Verstärkers (A1) bei der vorhandenen anorganischen Kohlendioxidkonzentration und gleichzeitig die Empfindlichkeit des zweiten Verstärkers (A2) für die Stickoxidkonzentration eingestellt wird und
- nach Beendigung der Einstellvorgänge für den Nullpunkt und für die Empfindlichkeit des NDIR-Gasanalysators das Meßgas (M) der Meßkammer (MK) und das Vergleichsgas (V) der Vergleichskammer (VK) zugeführt werden.

2. Verfahren nach Anspruch 1, dadurch gekennzeichnet, daß

zur kontinuierlichen Berücksichtigung des anorganischen Kohlendioxidanteils in der Gasprobe die Schritte zur Einstellung des Nullpunktes und der Empfindlichkeit des Gasanalysators in vorgegebenen zeitlichen Abständen wiederholt werden.

3. Verfahren nach Anspruch 1, dadurch gekennzeichnet, daß
- bei Betätigung eines ersten Ventils (MV1) in der Rückleitung von der Vergleichskammer (VK) zu dem thermischen Reaktor (OX) eine Verbindung zur Meßküvette (MV) hergestellt wird so, daß das Vergleichsgas (V) durch die Vergleichsküvette (VK) und durch die Meßküvette (MK) strömt,
- bei Betätigung eines zweiten Ventils (MV2) in der Zuleitung des Meßgases (M) zur Meßküvette (MV) vor oder hinter dem Kühler (K) ein Ablaß des Meßgases (M) aus dem Reaktor (OX) geschaffen wird und
- die Betätigung der Ventile (MV1, MV2) während der Einstellung des Nullpunktes und der Empfindlichkeit des Gasanalysators erfolgt.

4. Verfahren nach Anspruch 1 mit einer Kalibrierflüssigkeit mit Inhaltsstoffen für TOC und TN als Probe zur Kalibrierung der gesamten Vorrichtung, gekennzeichnet durch die folgenden Kalibrierschritte

- Einstellung des Nullpunktes und der Empfindlichkeit der Verstärker (A1,A2) entsprechend den letzten beiden Merkmalen des Anspruches 1 und

- Deaktivierung der Ventile (MV1,MV2) und Einstellung der Empfindlichkeit der Verstärker (A1,A2) auf die durch die Kalibrierflüssigkeit vorgegeben Konzentrationswerte für TOC und TN.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | DE-A-2 621 616 (SUMITOMO CHEMICAL CO., LTD.)<br>* das ganze Dokument *<br>--- | 1 | G01N33/18<br>G01N21/37 |
| A,D | DE-A-2 458 143 (ASTRO ECOLOGY CORP.)<br>* Abbildung 1 *<br>--- | 1 | |
| A,D, P | DE-A-3 937 141 (HARTMANN & BRAUN  AG)<br><br>* Zusammenfassung; Abbildung 2 *<br>--- | 1 | |
| A | DE-A-3 522 949 (HARTMANN & BRAUN AG)<br>* Zusammenfassung; Abbildung 1 *<br>--- | 1 | |
| A | DE-A-3 026 953 (HORIBA LTD.)<br>* Abbildung 3 *<br>--- | 1 | |
| A,D | DE-A-3 909 240 (HÜLS AG)<br>* Zusammenfassung *<br><br>----- | 1 | |
| | | | **RECHERCHIERTE SACHGEBIETE (Int. Cl.5)**<br><br>G01N |

Der vorliegende Recherchenbericht wurde für alle Patentansprüche erstellt

| Recherchenort | Abschlußdatum der Recherche | Prüfer |
|---|---|---|
| BERLIN | 18 AUGUST 1992 | BRISON O.P. |